# EUROPEAN PATENT SPECIFICATION

(11) **EP 3 515 474 B1**
(45) Date of publication and mention of the grant of the patent: **04.03.2026**
(21) Application number: 17771739.4
(22) Date of filing: 19.09.2017
(51) Int. Cl.: A61K 38/50, A61K 47/69, A61K 38/48, C12N 9/48, C12N 9/82, A61K 35/12, A61K 35/30, A61P 1/16, A61P 35/00

(54) **HUMAN ASPARAGINASE LACKING GLUTAMINASE ACTIVITY**
MENSCHLICHE ASPARAGINASE OHNE GLUTAMINASE-AKTIVITÄT
ASPARAGINASE HUMAINE DÉPOURVUE D'ACTIVITÉ GLUTAMINASE

(30) Priority: 19.09.2016 EP 16189525; 03.07.2017 EP 17179304
(43) Date of publication of application: 31.07.2019
(73) Proprietor: Cambridge Innovation Technologies Consulting Limited, Cambridge, Cambridgeshire CB4 0WS (GB)
(72) Inventor: PLUCHINO, Stefano, Cambridge Cambridgeshire CB4 2ZA (GB); FREZZA, Christian, Great Shelford Cambridgeshire CB22 5AE (GB); GAUDE, Edoardo, Cambridge Cambridgeshire CB1 3DE (GB); LEONARDI, Tommaso, Cambridge Cambridgeshire CB1 2QA (GB); IRACI, Nunzio, 98054 Furnari (IT)
(74) Representative: V.O.
(86) International application number: PCT/EP2017/073635
(87) International publication number: WO 2018/050918

(56) References cited:
- PIETERS ROB ET AL: "L-asparaginase treatment bin acute lymphoblastic leukemia", CANCER, vol. 117, no. 2, 15 January 2011 (2011-01-15), pages 238 - 249, XP002775935, DOI: 10.1002/cncr.25489
- DUVAL MICHEL ET AL: "Comparison of Escherichia coli-asparaginase with Erwinia-asparaginase in the treatment of childhood lymphoid malignancies: results of a randomized European Organisation for Research and Treatment of Cancer-Children's Leukemia Group phase 3 trial", BLOOD, AMERICAN SOCIETY OF HEMATOLOGY, US, vol. 99, no. 8, 15 April 2002 (2002-04-15), pages 2734 - 2739, XP002587208, ISSN: 0006-4971
- JASON R. CANTOR ET AL: "The Human Asparaginase-like Protein 1 hASRGL1 Is an Ntn Hydrolase with [beta]-Aspartyl Peptidase Activity", BIOCHEMISTRY, vol. 48, no. 46, 24 November 2009 (2009-11-24), US, pages 11026 - 11031, XP055289422, ISSN: 0006-2960, DOI: 10.1021/bi901397h
- AVRAMIS VASSILIOS I ET AL: "A randomized comparison of native Escherichia coli asparaginase and polyethylene glycol conjugated asparaginase for treatment of children with newly diagnosed standard-risk acute lymphoblastic leukemia: a Children's Cancer Group study", BLOOD, AMERICAN SOCIETY OF HEMATOLOGY, US, vol. 99, no. 6, 15 March 2002 (2002-03-15), pages 1986 - 1994, XP002523662, ISSN: 0006-4971, DOI: 10.1182/BLOOD.V99.6.1986
- OLLENSCHLAGER G ET AL: "Asparaginase-induced derangements of glutamine metabolism: The pathogenetic basis for some drug-related side-effects", EUROPEAN JOURNAL OF CLINICAL INVESTIGATION 1988 GB, vol. 18, no. 5, 1988, pages 512 - 516, XP002775936, ISSN: 0014-2972
- EVTIMOVA V ET AL: "IDENTIFICATION OF CRASH, A GENE DEREGULATED IN GYNECOLOGICAL TUMORS", INTERNATIONAL JOURNAL OF ONCO, DEMETRIOS A. SPANDIDOS ED. & PUB, GR, vol. 24, no. 1, 1 January 2004 (2004-01-01), pages 33 - 41, XP009038071, ISSN: 1019-6439
- KOICHI SUGIMOTO ET AL: "A clinically attainable dose of L-asparaginase targets glutamine addiction in lymphoid cell lines", CANCER SCIENCE, vol. 106, no. 11, 16 October 2015 (2015-10-16), JP, pages 1534 - 1543, XP055324570, ISSN: 1347-9032, DOI: 10.1111/cas.12807
- MOGHRABI ALBERT ET AL: "Results of the Dana-Farber Cancer Institute ALL Consortium Protocol 95-01 for children with acute lymphoblastic leukemia", BLOOD, vol. 109, no. 3, February 2007 (2007-02-01), pages 896 - 904, XP002775937
- KARPEL-MASSLER GEORG ET AL: "Metabolic reprogramming of glioblastoma cells by L-asparaginase sensitizes for apoptosis in vitro and in vivo", ONCOTARGET, vol. 7, no. 23, June 2016 (2016-06-01), pages 33512 - 33528, XP002775938
- IRACI NUNZIO ET AL: "Extracellular vesicles are independent metabolic units with asparaginase activity.", NATURE CHEMICAL BIOLOGY SEP 2017, vol. 13, no. 9, 3 July 2017 (2017-07-03), pages 951 - 955, XP002775939, ISSN: 1552-4469

## Description

### FIELD

The invention relates to the field of medicaments. The invention in particular relates to means and methods for treating individuals that suffer or are at risk of suffering from a malignancy. More specifically, the invention relates to a human asparaginase that can be used in a method for treating an individual suffering from a malignancy.

### 1 BACKGROUND OF THE INVENTION

L-asparaginase (EC 3.5.1.1), is an amidohydrolase that catalyzes the conversion of L-asparagine into L-aspartic acid and ammonia. L-asparaginase is known to have therapeutic value in the treatment of cancer, especially of leukemia (Sugimoto et al., 2015. Cancer Sci 106: 1534-1543). It is now well established that the inhibitory action of the enzyme is caused by the depletion of circulatory L-asparagine, which appears to be an essential nutrient for tumor cells. The administration of L-asparaginase into leukemic patients induces the selective death of the tumor cells by hydrolyzing L-asparagine, resulting in long-term event-free survival rates of around 80%, when combined with other antineoplastic agents (Moghrabi et al., 2007. Blood 109, 896-904).

Three types of asparaginase have been used until now: native asparaginase derived from *Escherichia coli;* an enzyme isolated from *Erwinia chrysantherraε,* and a pegylated form of the *E*. *coli* asparaginase (Pieters et al., 2011. Cancer 117, 238-49). Moreover, several alternative human asparaginases have been described, such as a human asparaginase lacking b-aspartyl peptidase activity and a human asparaginase named CRASH, that has a homology level of 32% compared to human aspartylglucosaminidase (Cantor et al., 2009. Biochemistry 48: 11026-11031; Evtimova et al., 2004. J Oncol 24: 33-41).

Clinical experience has shown that the administration of bacterial asparaginase may cause an immune responses ranging from systemic anaphylaxis to localised erythema and pain at the injection site (Miller and Boos, 1998. Critical Reviews in Oncology:Hematology 28, 97-113). Since asparaginases from different bacterial strains are available, treatment continuation in the event of immunological reactions to one asparaginase preparation may be possible by selecting an enzyme from a different biological source. In addition, pegylated forms may suitable be used in patients with hypersensitivity to the native form of a bacterial asparaginase (Avramis et al., 2009. Blood 99: 1986-1994).

In addition to elucidating an immune response, the presently used bacterial asparaginases have intrinsic glutaminase activity, resulting in severe toxicity of, for example, liver and pancreas (Duval et al., 2002. Blood 99, 2734-9).

### 2 SUMMARY OF THE INVENTION

Various bacteria-derived asparaginases are currently used to treat acute lymphoblastic leukaemia and malignant glial neoplasms (Karpel-Massler et al., 2016. Oncotarget 7, 33512-33528), but their residual glutaminase activity is thought to be a major drawback of therapy since it leads to secondary immune depression and liver toxicity (Ollenschlager et al., 1988. Eur J Clin Invest 18, 512-516). Therefore, the identification of glutaminase-free functional L-asparaginase might provide a valid alternative to recombinant L-asparaginases that would limit their side effects.

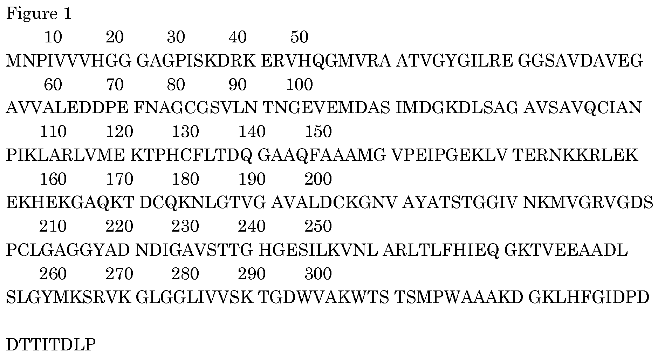

The present invention therefore provides a human asparaginase comprising an amino acid sequence that is at least 90% identical to the amino acid sequence depicted in Figure 1, for use as a medicament and wherein the asparaginase lacks glutaminase activity. Said asparaginase was found to lack intrinsic glutaminase activity and thus provides a safe alternative to recombinant bacterial L-asparaginases. In addition, a human protein will likely result in a reduced allergenic response in humans, compared to the bacterial enzymes.

Said asparaginase for use according to the invention preferably is in a method for treating a malignancy, preferably a blood cell malignancy, or in a method for metabolic reprogramming of a cell, preferably a cancerous cell. Inhibition of several of the key enzymes involved in metabolic pathways has been demonstrated to effectively obstruct cancer cell growth and multiplication, sensitizing them to apoptosis. The goal of treating cancer by altering tumor metabolism has gained much attention, and several products are now being tested in the clinic (Garbor, 2016. Nature Biotech 34: 794-795; Karpel-Massler et al., 2016. Oncotarget 7, 33512-33528).

It is preferred that an asparaginase according to the invention is provided in a membranous particle, preferably a cell such as a human cell, preferably a human stem cell, or in a naturally occurring extracellular membrane vesicle (EV) or an artificial liposome.

As an alternative, said asparaginase is recombinantly produced in a bacterium, in a yeast, or in a human cell, preferably wherein a nucleotide sequence that encodes said asparaginase is codon-optimized for expression in said bacterium or yeast.

In one embodiment, said asparaginase is pegylated to increase the hydrodynamic size (size in solution) of the agent which prolongs its circulatory time by reducing renal clearance.

An asparaginase for use according to the invention is preferably for use in a method for treatment of a patient suffering from leukemia, myelodysplastic syndrome, or a solid tumor such as hepatocellular carcinoma, renal cell carcinoma, breast cancer, ovarian cancer, brain tumor, or lung cancer, especially of a patient suffering from acute lymphocytic leukemia or acute myeloid leukemia.

An asparaginase for use according the invention is preferably provided to a patient that had previously been treated with an asparaginase from *Escherichia coli* or from *Erwinia chrysanthemi,* especially a patient that is suffering from severe toxicity such as liver toxicity.

An asparaginase for use according to invention is preferably provided as part of a therapy (adjuvant) that further comprises at least one antineoplastic agent. Depending on the tumor, said at least one antineoplastic or pro-apoptotic agent is preferably selected from vincristine, mercaptopurine, methotrexate, daunorubicin, navitoclax/obatoclax and prednisone.

In one embodiment, said asparaginase for use as a medicament may be provided in a vector, preferably a viral vector, for transduction of tumor cells and local expression of said asparaginase. Said vector preferably is targeted to the tumor cells and/or said asparaginase is conditionally expressed in tumor cells.

The invention further provides a pharmaceutical composition, comprising an asparaginase comprising an amino acid sequence that is at least 90% identical to the amino acid sequence depicted in Figure 1 and wherein the asparaginase lacks glutaminase activity, and a pharmaceutically acceptable excipient.

Further described herein is an asparaginase for use as a medicament, wherein the asparaginase is provided in a membranous particle, preferably a cell, more preferably a human cell such as a human stem cell, or a vesicle, preferably a naturally occurring extracellular membrane vesicle (EV), said asparaginase having an amino acid sequence that is at least 90% identical to the amino acid sequence depicted in Figure 1 and wherein the asparaginase lacks glutaminase activity. Said particle preferably is an EV obtained from a stem cell, preferably from a human neural stem cell.

The invention further provides a pharmaceutical composition comprising a particle as described herein and a pharmaceutically acceptable excipient.

### 3 FIGURE LEGENDS

Figure 1. Amino acid sequence of human asparaginase-like 1.
Figure 2. Neural stem cell (NSC) extracellular membrane vesicles (EVs) are metabolically active in vitro
   (A) Schematic representation of the metabolomic experiment in medium + EVs *us.* fresh medium (Vehicle). (B) Scatter plot of metabolomics experiment showing log₂ fold changes of extracellular metabolites in medium + EVs *vs.* Vehicle as in A. Positive/negative values indicate production/consumption of metabolites, respectively. Metabolites that were significantly (p< 0.05) consumed or produced are highlighted in black. Data from one representative experiment is shown. Statistical analysis was performed using Student's t test followed by Benjamini-Hochberg correction. (C) Barplot of the consumption of ¹⁵N₂-Asn and production of ¹⁵N-Asp mediated by EVs, conditioned medium (i.e. CM, medium with EVs) and supernatant (i.e. SN, medium deprived of EVs), with or without heat inactivation (100°C for 10'). Data are mean ± SEM and have been obtained from n= 2 independent experiments. Statistical analysis was performed using one-way ANOVA, followed by Bonferroni's test correction. * p< 0.05; ** p < 0.01; *** p<0.001. (D) Consumption of Asn with different amounts of clinical-grade asparaginase produced by *Erwinia chrysanthemi* (Erwinase) was used to determine the asparaginase activity associated with EVs. One International Unit of asparaginase is defined as the amount of enzyme required to generate 1 µmol of ammonia per minute at pH 7.3 and 37°C. Solid line indicates Erwinase calibration curve, while square symbol and dashed line indicate interpolation of the calibration curve with Asn consumption values obtained with EVs.

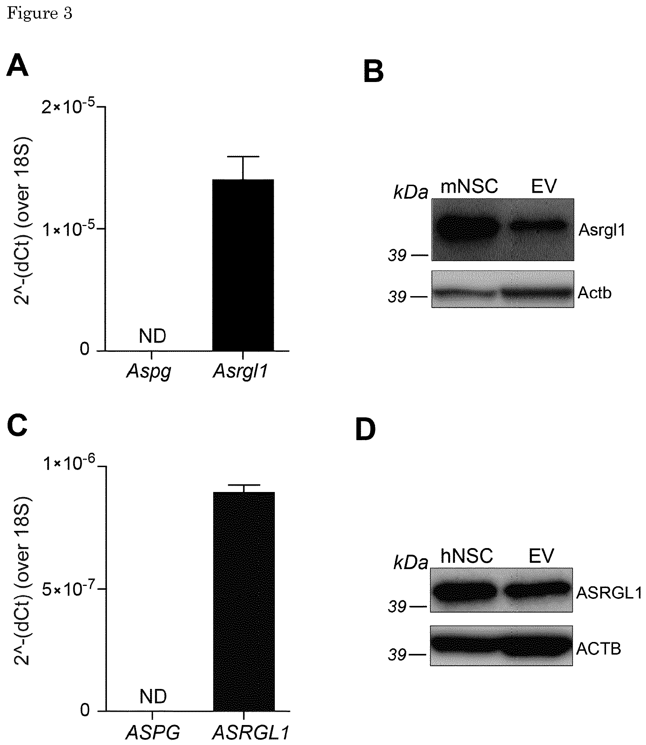

Figure 3. Mouse and human NSCs traffic Asrgl1 into EVs
   (A) quantitative polymerase chain reaction (qPCR) and (B) Western blot (WB) analyses of Asrgl1 expression in mouse NSCs. qPCR data are represented as fold change ± standard error of the mean (SEM) of the *Aspg* and *Asrgl*1 mRNA levels in mouse NSCs, and analysed with the 2^{-ΔCt} method (Ginzinger, 2002. Experimental Hematology 30, 503-512) over 18S ribosomal RNA, used as housekeeping gene. Both qPCR and WB data have been obtained from n=3 independent experiments. (C-D) Same as in A-B for human neural stem cells (hNSCs).

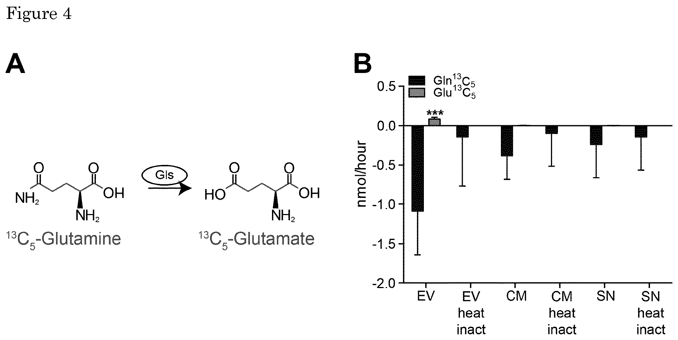

Figure 4. The asparaginase activity of EVs is devoid of glutaminase activity (A) Schematic representation of isotope tracing experiment. Reaction carried out by glutaminase (Gls). (B) Barplot of the consumption of ¹³C₅-Gln and production of ¹³C₅-Glu mediated by EVs, conditioned medium (i.e. CM, medium with EVs) and supernatant (i.e. SN, medium deprived of EVs), with or without heat inactivation (100°C for 10'). Data are mean ± SEM and have been obtained from n= 2 independent experiments. Statistical analysis was performed using one-way ANOVA, followed by Bonferroni's test correction. *** p<0.001.

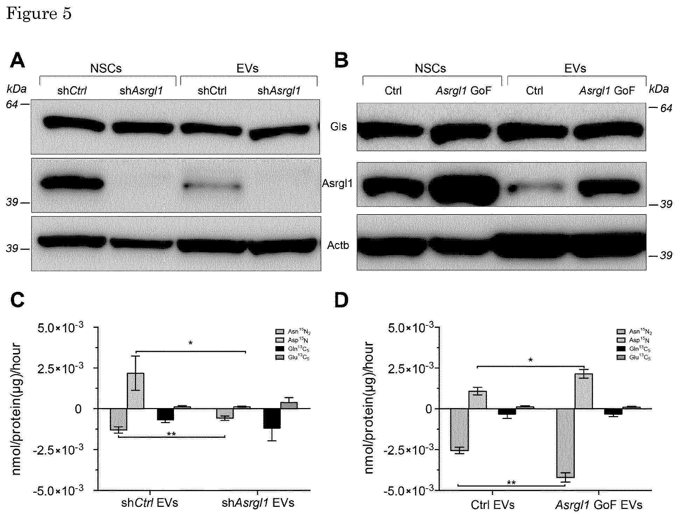

Figure 5. Asrgl1 is responsible for the selective asparaginase activity associated with EVs
   (A-B) Western blot analysis of shAsrgl1 (A) and Asrgl1 Gain of Function (GoF) (B) NSCs and EVs. These panels are representative of n= 3 independent protein preparations showing the same trends. (C-D) Barplot of the consumption of ¹⁵N2-Asn and ¹³C5-Gln followed by production of ¹⁵N-Asp and ¹³C5-Glu mediated by shAsrgl1 (C) and Asrgl1 GoF (D) NSC EVs. Data are mean ± SEM and have been obtained from n= 3 independent experiments. Statistical analysis was performed using t-test. * p< 0.05; ** p < 0.01.

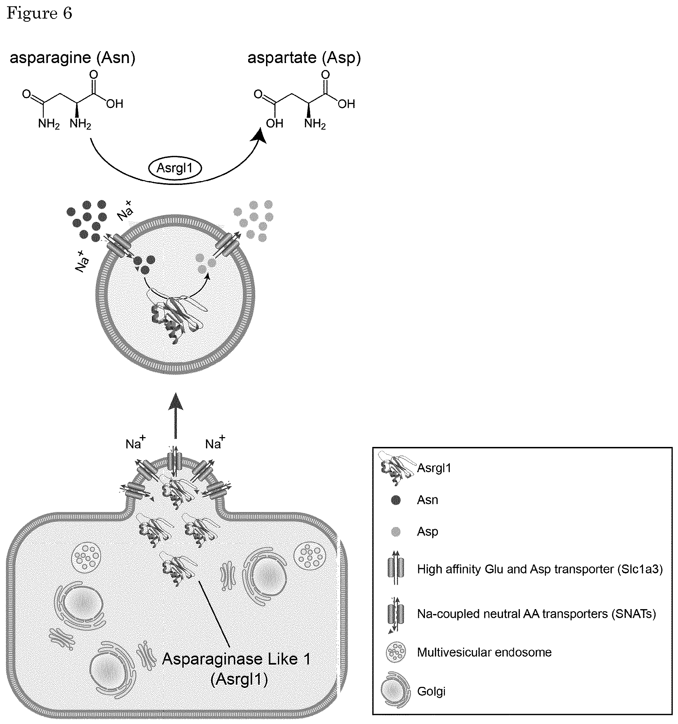

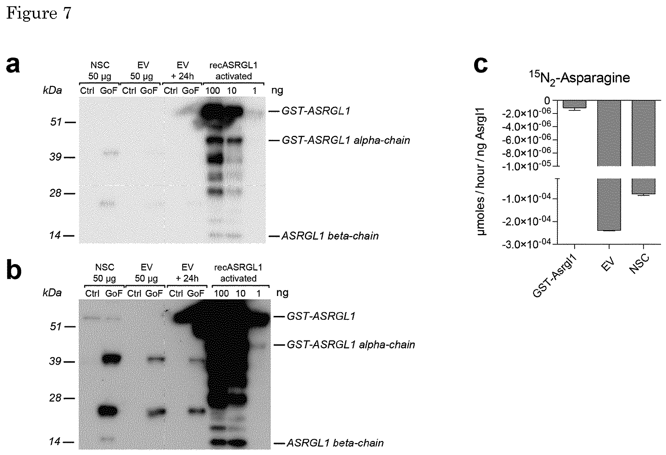

Figure 6. Schematic representation of putative mechanism of action of asparaginase-like 1 containing NSC-EVs.

### 4 DETAILED DESCRIPTION OF THE INVENTION

### 4.1 Definitions

The term asparaginase, or L-asparaginase, as is used herein, refers to an enzyme that catalyzes the conversion of L-asparagine into L-aspartic acid and ammonia. Most asparaginases also possess glutaminase activity. A preferred L-asparaginase is human isoaspartyl peptidase/L-asparaginase or asparaginase-like 1 (ASRGL1). The native enzyme, depicted in Figure 1, encompasses 308 amino acids, which are cleaved into an alpha and beta chain by autocatalysis upon activation of the enzyme.

The term identity, or identical, as is used herein, refers to the percentage of amino acid residues that are identical between two amino acid sequences when these amino acid sequences are aligned for maximum correspondence. Sequence comparison between two or more amino acid sequences is generally performed by comparing portions of the two sequences over a comparison window to identify and compare local regions of sequence similarity. The identity of two proteins or peptides, as provided by the % of identical amino acid residues, is determined over the whole protein or peptide. For example, a protein that is at least 90% identical to a protein with a length of 100 amino acid residues has more than 90% identical amino acids at identical positions when the complete two amino acid sequences are aligned for maximum correspondence.

The term recombinantly expressed, as is used herein, refers to the expression of a cDNA molecule that encodes an amino acid sequence that is at least 90% identical to the amino acid sequence depicted in Figure 1 in a cell using molecular biology techniques. Said cell preferably is a bacterial cell, for example *Escherichia coli,* or a yeast cell such as, for example *Saccharomyces cerevisiae* or *Pichia pastoris.* A preferred cell is a human cell or cell line, which have specific cellular machinery and characteristic mechanisms for post-translational activities (e.g., HeLa, HEK293, PER.C6, and WI38). Said cells are available from the American Type Culture Collection (ATCC; 10801 University Boulevard, Manassas, VA 20110-2209) and can be chosen to ensure the correct modification and processing of a recombinantly expressed asparaginase.

The term pegylated, as is used herein, refers to the covalently linking of polyethylene glycol (PEG) to the asparaginase as described herein. The PEG is preferably linked to one or more lysine residues and/or the N-terminus of the L-asparaginase. Said PEG is preferably linked to at least from about 40% to about 100% of the accessible lysine residues and/or the N-terminus of the protein. Said PEG preferably has an average molecular weight between about 1000 and 100,000 daltons, more preferably between 4000 and 40,000 daltons.

The term liposome, as is used herein, refers to an encapsulating agent, preferably an artificial or man-made agent, that comprises at least one closed lipid bilayer such as a membrane, which defines an aqueous compartment encompassing the asparaginase. A liposome may be formed by a lipid or combination of lipids, including but not limited to, polar lipids, such as phospholipids, phosphoglycerides, phosphatidylethanolamine, phophatidylcholine, phosphatidylserine, cardiolipin or combinations thereof. Other lipid moieties may also be included in the liposomes such as triacylglycerols, waxes, sphingolipids, and sterols and their fatty acid esters, or combinations thereof.

The term "membranous particle" as is used herein, refers to any particle that is surrounded by a lipid layer, preferably a cellular membrane. A membranous particle preferably is a cell, more preferably a human cell such as a human stem cell, a vesicle, preferably a naturally occurring extracellular membrane vesicle (EV), or a liposome.

The term extracellular membrane vesicles (EVs), as is used herein, refers to spherical structures that are limited by a lipid bilayer and that contain hydrophilic soluble components. Cells produce many EVs regulating the transfer of components between intracellular compartments, but it is now clear that many eukaryotic cells generate membrane vesicles that are secreted into the extracellular space, and are therefore potential carriers for intercellular communication. EVs can form at the plasma membrane (*membrane particles*) by direct budding or shedding into the extracellular space, giving rise to large-size (> 100 nm) membrane particles, also defined as microvesicles, ectosomes, microparticles or exovesicles (Thery et al., 2009. Nat Rev Immunol 9: 581-93). Alternatively, secreted EVs can form internal compartments (late endocytic) from where they are subsequently secreted by fusion with the plasma membrane. The recycling pathways of endocytosed materials and components of the cell surface involve several sorting events, which are regulated by molecular motors and take place at different steps of the pathways. The EVs generated in multivesicular endosomes (*exosomes*) are small in size (40-100 nm), once secreted, and the exact nature of the intra cellular compartments from which exosomes derive is still unclear. Finally, also exosome-like vesicles (20-50 nm) may originate from multivesicular internal compartments (not necessarily being part of the endosomal system), and their nature is not completely clear. Irrespective of their origin, all these vesicles contain cytosol and expose on their outer surface the extracellular side of the membrane from which they are formed (*flip-flop* mechanism). EVs are also released by dying and/or apoptotic cells. Large membrane particles and ectosomes have high levels of phosphatidylserine exposed on the outer membrane - usually depending on cell type and stimuli - and express the complement receptor (CR)-1, whereas exosomes are enriched in tetraspanins (eg., CD63, CD9) Maxfield, F.R. and T.E. McGraw, Endocytic recycling. Nat Rev Mol Cell Biol, 2004. 5(2): p. 121-32).

The term antineoplastic agent, as is used herein, refers to an agent that is useful in cancer therapy against a wide array of cancer. Non-limiting examples of useful antineoplastic agents include temozolomide, uracil mustard, chlormethine, cyclophosphamide, ifosfamide, melphalan, chlorambucil, pipobroman, triethylenemelamine, triethylenethiophosphoramine, busulfan, carmustine, lomustine, streptozocin, dacarbazine, methotrexate, 5-fluorouracil, floxuridine, cytarabine, 6-mercaptopurine, 6-thioguanine, fludarabine phosphate, pentostatine, gemcitabine, vinblastine, vincristine, vindesine, bleomycin, dactinomycin, daunorubicin, doxorubicin, epirubicin, idarubicin, paclitaxel, mithramycin, deoxycoformycin, mitomycin-c, interferons, etoposide, teniposide 17.alpha.-ethinylestradiol, diethylstilbestrol, testosterone, prednisone, fluoxymesterone, dromostanolone propionate, testolactone, megestrolacetate, tamoxifen, methylprednisolone, methyltestosterone, prednisolone, triamcinolone, chlorotrianisene, hydroxyprogesterone, aminoglutethimide, estramustine, medroxyprogesteroneacetate, leuprolide, flutamide, toremifene, goserelin, cisplatin, carboplatin, hydroxyurea, amsacrine, procarbazine, mitotane, mitoxantrone, levamisole, navelbene, anastrazole, letrazole, capecitabine, reloxafine, droloxafine, hexamethylmelamine, and mixtures thereof. A preferred antineoplastic agent is or comprises vincristine, mercaptopurine, methotrexate, daunorubicin, navitoclax/obatoclax and prednisone.

The term pharmaceutically acceptable excipient, as is used herein, refers to excipients that are required to formulate a dosage form such as but not limited to preservatives, polymers, and wetting agents. Suitable preservatives include, but are not limited to, benzalkonium chloride, benzethonium chloride and cetyl pyridinium chloride, benzyl bromide, benzyl alcohol, disodium EDTA, phenylmercury nitrate, phenylmercury acetate, thimerosal, merthiolate, acetate and phenylmercury borate, polymyxin B sulphate, chlorhexidine, methyl and propyl parabens, phenylethyl alcohol, quaternary ammonium chloride, sodium benzoate, sodium propionate, stabilized oxychloro complex, and sorbic acid. Examples of suitable polymers include, but are not limited to: cellulose derivatives such as methylcellulose, hydroxyethylcellulose, hydroxypropylcellulose; polyvinylpyrrolidone and polyvinylalcohol or mixtures thereof. Examples of suitable wetting agents include, but are not limited to: polyoxyethylene, sorbitan monolaurate and stearate.

The term stem cell (SC), as is used herein, refers to an undifferentiated cell that can proliferate, self-renew, and differentiate into the specialized cells. Preferred stem cells are embryonic stem cells, tissue-specific stem cells such as hematopoietic stem cells, liver stem cells, mesenchymal stem cells and NSCs. A most preferred stem cell is a NSC.

As used herein, the terms "treatment," "treating," and the like, refer to obtaining a desired pharmacologic and/or physiologic effect. The effect may be therapeutic in terms of a partial or complete cure for a disease and/or adverse affect attributable to the disease. "

### 4.2 Membranous particle comprising human asparaginase

The invention provides an asparaginase comprising an amino acid sequence that is at least 90% identical to the amino acid sequence depicted in Figure 1, for use as a medicament and wherein the asparaginase lacks glutaminase activity.

Said asparaginase lacking glutaminase activity is preferably provided in a membranous particle. Said membranous particle may be a cell, preferably a stem cell, or a vesicle, preferably a EV. The presence in a membranous particle might protect the enzyme from degradation, thereby providing a longer lasting medical use of said asparaginase.

In an embodiment, said asparaginase lacking glutaminase activity is provided in a cell, preferably a human neural stem cell (hNSC). A cell such as a neural stem cell produces endogenous L-asparaginase comprising an amino acid sequence that is at least 90% identical, preferably at least 95%, more preferably at least 99% identical, to the amino acid sequence depicted in Figure 1 and wherein the asparaginase lacks glutaminase activity.

This enzyme, also termed asparaginase-like 1 (ASRGL1), lacks intrinsic glutaminase activity and is therefore extremely well suited for use as a medicament according to the invention. Furthermore, said human enzyme will likely be less immunogenic when applied to human, when compared to the bacterial asparaginase molecules that are presently used.

Methods for isolation of stem cells are known in the art. For example, embryonic stem (ES) cells can be obtained from the inner cell mass of a blastocyst, for example from blastocysts created by *in vitro* fertilization that are no longer needed. Tissue-specific stem cells, also referred to as somatic or adult stem cells, can be isolated by fragmentation of tissue and growing cells that are obtained in specialized growing supplemented with specific growth factors such as basic fibroblast growth factor, epidermal growth factor and leukemia inhibitory factor. Mesenchymal stem cells, also termed "stromal cells", can be isolated from bone marrow, fat and cord blood.

A skilled artisan will appreciate that also stem cell lines can be used as a source of asparaginase-like 1 (ASRGL1). Hence, an asparaginase comprising an amino acid sequence that is at least 90% identical to the amino acid sequence depicted in Figure 1, can be provided in a cell of a human stem cell line, including an embryonic stem cell line, for example as described in The International Stem Cell Initiative, 2007. Nature Biotechnology 25, 803 - 816) and a neural stem cell line (Donato et al., 2007. BMC Neuroscience 8, 36).

Some stem cell enrichment protocols rely on sets of antibodies to cell surface proteins and employ methods to separate cells that react with the antibodies from cells that do not react, for example by employing a fluorescence-activated cell sorter. An alternative method for identifying stem cells may involve the efflux of the DNA binding dye, Hoechst 33342, (Dunnwald et al., 2001. Exp Dermat 10: 45).

Human NSCs can be derived from human ES cells, or human somatic cells via human induced pluripotent stem cells (iPSCs) and the differentiation into NSCs or direct cell conversion into stably expandable human iNSCs (Schuldiner et al., 2001. Brain Res 913, 201-5; Li and Zhang, 2006. Methods Mol Biol 331, 169-77; Shin et al., 2007. Stem Cells 25, 1298-306; Wen and Jin, 2014. J Biotechnol 188, 122-9; Lie et al., 2012. Methods Mol Biol 873, 237-46).

Methods for isolation of human NSC are known in the art. For example, foetal human NSCs can be isolated from natural in utero death. For example, forebrain human fetal brain tissue specimens may be collected from fetuses at gestational ages greater than the 8th post-conception week to generate hNSC lines as decribed (Vescovi et al., 1999. Exp Neurol 156, 71-83; Mazzini et al., 2015. Journal of Translational Medicine 13, 17). Cultures can be maintained in a humidified incubator at 37°C, 5% O2 and 5% CO2 and be allowed to proliferate as free-floating clusters (neurospheres). 7-10 days after the primary cell seeding the neurospheres may be collected and mechanically dissociated and replated at the same initial density. This step (passaging) may be routinely repeated up to >20 times. Throughout these passages aliquots of cells may be frozen as neurospheres, and cryopreserved in 10% dimethyl sulfoxide as a pharmaceutical intermediate product, with an assigned batch number. It may also be allowed for the establishment of an "Intermediate Product" to warrant the series of quality controls required to certify the safety, identity, potency and the pharmaceutical grade of the donor hNSCs according to regulatory requirements.

In an embodiment, said asparaginase lacking glutaminase activity is provided in a vesicle, preferably an extracellular membrane vesicle (EV).

Methods for isolating EVs are known in the art. For example, EVs can be isolated from stem cells, such as human stem cells, preferably human NSCs, by centrifugation, for example by three successive centrifugations at 300 g (5 min), 1,200 g (20 min), and 10,000 g (30 min) to eliminate cells and debris, followed by centrifugation for 1 h at 110,000 g, as described (Théry et al., 1999. J Cell Biol 147, 599-610). The exosome pellet may be washed once in a large volume of PBS, centrifuged at 110,000 g for 1 h, and resuspended in 50-200 ml of PBS with 0.01% sodium azide. As an alternative, flotation of exosomes on a continuous sucrose gradient may be performed as described (Raposo et al., 1996. J Exp Med 183, 1-12). Alternative methods are known to the skilled person and are described, for example, in Witwer et al., 2013. J Extracell Vesicles 27, 20360.

A preferred cell for release of a vesicle comprising an asparaginase as described herein is a stem cell, preferably a neural stem cell (NSC), also termed neural precursor cell.

The human asparaginase-like 1 (ASRGL1) that is present in the EVs, lacks intrinsic glutaminase activity. A glutaminase activity that was found to be associated with the EVs, is likely a contamination from the mitochondria of lysed cells and can be purified away from the EVs.

As is reported herein above, asparaginase-like 1 lacks intrinsic glutaminase activity and is likely to be less immunogenic when applied to human, when compared to the bacterial asparaginase molecules that are presently used. A further advantage of EVs comprising ASRGL1 is that these vesicles comprises asparagine transporters such as members of the solute carrier (SLC) family of membrane transporters, which include the high affinity Glu and Asp transporter (Slc1a3) and the two system A family members the Sodium-coupled neutral amino acid transporter 2 (Slc38A2), and the Sodium-coupled neutral amino acid transporter 3 (Slc38A3) (Cossetti et al., 2014. Mol Cell 56, 193-204). Metabolically active EVs may inwardly transport extracellular asparagine via sodium-coupled neutral amino acid (system N/A) transporters, hydrolyze asparagine to aspartate via the internally present ASRGL1, and then export aspartate in the extracellular milieu (see Figure 6). In this way, an effective, shielded, all human, autonomous unit provides the efficient turnover from extracellular asparagine into aspartic acid.

The finding that EVs-derived Asrgl1 is specific for Asn has important clinical implications. Various bacteria-derived asparaginases are currently used to treat acute lymphoblastic leukaemia, brain tumors (Panosyan et al., 2014. Mol Cancer Res 12: 694-702), and malignant glial neoplasms (Karpel-Massler et al., 2016. Oncotarget 7, 33512-33528; Agnihotri and Zadeh 2016. Neuro Oncol 18: 160-72), but their residual Gls activity is thought to be a major drawback of therapy since it leads to secondary immune depression and liver toxicity (Ollenschlager et al., 1988. Eur J Clin Invest 18, 512-516). Therefore, the identification of glutaminase-free functional L-asparaginase in EVs might provide a valid alternative to recombinant L-asparaginases that would limit most of their side effects.

In an embodiment, said membranous particle is a liposome. A liposome may consist of single or multiple concentric lipid bilayers encapsulating an aqueous compartment encompassing the asparaginase. The lipid bilayers may be composed of natural lipids; synthetic lipids and mixtures thereof. Preferred liposomes have a diameter between 50 and 450 nm.

Said liposome preferably comprises amino acid transporters, preferably asparagine transporters such as members of the solute carrier (SLC) family of membrane transporters, which include the high affinity Glu and Asp transporter (Slc1a3) and the two system A family members the Sodium-coupled neutral amino acid transporter 2 (Slc38A2), and the Sodium-coupled neutral amino acid transporter 3 (Slc38A3) (Cossetti et al., 2014. Mol Cell 56, 193-204). Said liposome may preferably inwardly transport extracellular asparagine via sodium-coupled neutral amino acid (system N/A) transporters, hydrolyze asparagine to aspartate via the internally present ASRGL1, and then export aspartate in the extracellular milieu, similar to EV.

Methods for the preparation of liposomes are known in the art, including the thin-film hydration method, reverse-phase evaporation and solvent-injection methods, a detergent-depletion method, a heating method, spray-drying, freeze-drying, supercritical reverse-phase evaporation, and several modified ethanol-injection techniques, and a microfluidic-based method (Bozzuto and Molinari, 2015. Int J Nanomedicine. 10, 975-999).

For all embodiments described herein, it is preferred that all steps for the generation of an asparaginase comprising an amino acid sequence that is at least 90% identical to the amino acid sequence depicted in Figure 1 and wherein the asparaginase lacks glutaminase activity that is provided in a membranous particle are performed according to good manufacturing practice protocols, as dictated by the European Medical Agency guidelines.

### 4.3 Recombinant expression of L-asparaginase

In one embodiment, said asparaginase lacking glutaminase activity is produced in a bacterium, in a yeast, or in a human cell. The person skilled in the art will understand how to generate a DNA sequence that encodes said asparaginase using generally known recombinant DNA techniques. The sequence of the nucleic acid molecule is preferably codon-optimized for expression in a host cell, especially when expressed in a bacterium or yeast, for high-level expression in a such host cell.

An asparaginase-encoding DNA molecule can be made by a cell and isolated using standard nucleic acid purification techniques, or synthesized using an amplification technique, such as the polymerase chain reaction (PCR), or by using an automatic synthesizer. Methods for isolating DNA molecules are routine and are known in the art. Any such technique for obtaining a DNA molecule that can be used to obtain such asparaginase-encoding DNA.

A DNA sequence that encodes said asparaginase lacking glutaminase activity is preferably provided in an expression vector. For this, nucleic acid molecules are preferably inserted in an expression vector using recombinant DNA techniques known by the person skilled in the art. Expression vectors in the context of the invention direct the expression of a protein as described herein in a host cell. These expression vectors are preferably replicable in a host cell, either as episomes or as part of the chromosomal DNA. Further, the expression vector preferably comprises (i) a strong promoter/enhancer, such as the CMV or SV40 promoter, (ii) an optimal translation initiation sequence, such as a ribosomal binding site and start codon, preferably a KOZAK consensus sequence and (iii) a transcription termination sequence, including a poly(A) signal when the protein is expressed in eukaryotic cells. Suitable expression vectors include plasmids and viral vectors such as adenoviruses, adeno-associated viruses and retroviruses. The person skilled in the art will understand that the expression vector to be used is dependent on the host cell that is used for expression of a recombinant protein. An expression vector is preferably suited for expression of a nucleic acid molecule in a prokaryotic cell including a bacterial cell, or, more preferred, in a eukaryotic host cell, such as a yeast cell and a mammalian cell.

Said expression vector preferably further comprises a signal sequence which ensures that the recombinant asparaginase lacking glutaminase activity is secreted from the cell in which it is expressed. Said signal sequence can be a short (5-30 amino acids long) peptide present at the N-terminus of the asparaginase. A signal sequence is preferably cleaved upon secretion of the asparaginase from the cell. Suitable signal sequences are known in the art, for example as described in Kober et al., 2013. Biotechnol Bioeng 110: 1164-73.

As an alternative, a nucleic acid molecule as described herein may be inserted in the genome of a host cell. Said insertion preferably is at a locus or within a region that ensures expression of a nucleic acid molecule encoding said asparaginase in the host cell.

Suitable host cells include prokaryotic and eukaryotic cells, such as bacterial cells, yeast cells, insect cells, animal cells, mammalian cells, murine cells, rat cells, sheep cells, simian cells and human cells. Examples of suitable eukaryotic host cells include, but are not limited to HEK 293 cells, the hamster cell line CHO and BHK-21; the murine host cells NIH3T3, NSO and C127; the simian host cells COS and Vero; and the human host cells HeLa, PER.C6, U-937 and Hep G2. Suitable cells are available from public sources such as ATCC and Life Technologies. A number of transfection techniques are known in the art for introducing a nucleic acid molecule encoding said asparaginase in a host cell, see, e.g., Graham et al., 1973. Virology 52: 456; Green et al., 2012. "Molecular Cloning: A Laboratory Manual", CSHL Press; Davis et al., "Basic Methods in Molecular Biology", 1986, Elsevier; and Chu et al., 1981. Gene 13: 197. The person skilled in the art preferably employs techniques as described in these references to introduce one or more exogenous nucleic acid molecules into suitable host cells.

A particularly preferred host cell for the production of a protein as described herein is a human cell, including a cell of a cell line such as, for example, HEK 293, HeLa, PER.C6, WI38, U-937 and Hep G2.

Said asparaginase lacking glutaminase activity that is produced in a host cell is preferably purified from any cell which expresses the enzyme, including host cells, and is isolated free of other cellular components such as membrane components, proteins, and lipids using methods well-known in the art. Such methods include, but are not limited to, size exclusion chromatography, ammonium sulfate fractionation, ion exchange chromatography, affinity chromatography, and preparative gel electrophoresis. A preparation of purified -asparaginase is preferably at least 80% pure, more preferably, at least 90%, 95%, or 99% pure. Purity of the preparations can be assessed by any means known in the art, such as SDS-polyacrylamide gel electrophoresis.

A nucleic acid molecule encoding said asparaginase lacking glutaminase activity may be fused in frame to a nucleotide sequence encoding a polypeptide domain which will facilitate purification of the fusion protein. Such purification facilitating domain includes, but is not limited to, a metal chelating peptide such as histidine-tryptophan module that allows purification on immobilized metals, a glutathione-S transferase domain, a protein A domain that allows purification on immobilized immunoglobulin, and a domain such as FLAG-tag, HA-tag, or Myc-tag. A cleavable linker sequence my be included between the purification domain and the asparaginase ((Terpe, 2003. Appl Microbiol Biotechnol 60, 523-533).

A recombinant asparaginase lacking glutaminase activity that is produced in a host cell may be administered as purified protein to a person in need thereof.

In an embodiment, said asparaginase lacking glutaminase activity is pegylated after production in a host cell, and purification of the enzyme. Pegylation results in an increase of the molecular mass of the protein and may help to shield the protein from proteolytic enzymes. Typical reactive amino acids include lysine, cysteine, histidine, arginine, aspartic acid, glutamic acid, serine, threonine, tyrosine, and the N-terminal amino group and the C-terminal carboxylic acid. Preferred amino acids are lysine residues, and the N-terminal amino group. PEGylation of proteins can be achieved by a chemical reaction between an amino acid of the protein and a suitably activated PEGylation reagent. Examples of suitable PEGylation reagents include reactions of N-hydroxysuccinimidyl ester derivatized PEGs with lysine; 3 1,4-addition reactions of maleimide and vinylsulfone derivatized PEGs with cysteine, and condensation of hydrazide containing PEGs with aldehydes generated by oxidation of glycoproteins, as are known to the skilled person. Methods for site-specific incorporation of PEG residues are known (Deiters et al., 2004. Bioorganic Med Chem Lett 14, 5743-5745; Dozier and Distefano, 2015. Int. J. Mol. Sci.16, 25831-25864). Site-specific PEGylation may result in minimizing a decrease in activity associated with overall PEGylation while retaining the pharmacokinetic benefits that accompany PEG attachment.

As an alternative, said asparaginase lacking glutaminase activity may be produced by the subject itself following introduction of a nucleic acid molecule encoding said asparaginase lacking glutaminase activity into an expression vector, preferably a viral vector, preferably a viral vector with a target cell selectivity for a specific target cell such as a cancer cell originating from the hemopoietic system, a liver cancer cell, a pancreatic cancer cell, a, skin cancer cell, a gastrointestinal cancer cell, a kidney cancer cell, an ovarian cancer cell, a breast cancer cell, a prostate cancer cell, a brain cancer cell, or a head/neck cancer cell.

A variety of host-expression vector systems may be utilized to express an asparaginase molecule lacking glutaminase activity as defined herein. Suitable vectors include vectors based on gammaretrovirus, lentivirus, adenovirus (AdV), adeno-associated virus (AAV) and herpes simplex virus (HSV). Methods for targeting tumor cells are known in the art, including pseudotyping of retroviral vectors with a viral glycoprotein that binds to a specific membrane receptor of the tumor cell type. Furthermore, a viral glycoprotein can be fused with a ligand protein or antibody that recognizes cell type-specific surface molecules, providing a versatile way of cell type-specific gene delivery (Vannucci et al., 2013. New Microbiol 36, 1-22).

### 4.4 Administration of asparaginase.

An asparaginase lacking glutaminase activity for use according to the invention is for use as a medicament, preferably for treating a malignancy, preferably a B cell blood malignancy, or in a method for metabolic reprogramming of a cell, preferably a cancerous cell.

Said asparaginase lacking glutaminase activity for use according to the invention preferably is in a method for treatment of a patient suffering from leukemia, myelodysplastic syndrome, a solid tumor such as hepatocellular carcinoma, renal cell carcinoma, breast cancer, ovarian cancer, brain tumor or lung cancer. Said leukemia preferably is selected from acute lymphocytic leukemia and acute myeloid leukemia.

Said asparaginase lacking glutaminase activity preferably is administered by parenteral administration, which is selected from intravenous, intra-arterial, intramuscular, subcutaneous, intradermal, and intraperitoneal administration. Preferred routes for administering said asparaginase are intravenous or intramuscular injection, or intravenous infusion.

A skilled person will appreciate that for treatment of a brain tumor such as a glioma, including glioblastoma, astrocytoma, oligodendroglioma, and ependymoma, said asparaginase preferably is directly provided in the brain, such as by direct injection in situ or infusion into the brain.

Asparaginase lacking glutaminase activity for use according to the invention is preferably provided in a dosage of at least 6000 IU/square meter, preferably at least 10,000 IU/square meter, more preferably at least 20,000 IU/square meter. Administration preferably is at least once a week, more preferably three times weekly. One asparaginase unit (IU) is defined as the amount of enzyme that liberates 1 micro-mol per minute of ammonia under standard assay conditions.

Asparaginase lacking glutaminase activity for use according to the invention is preferably provided to a subject that had been treated with an asparaginase from *Escherichia coli* or from *Erwinia chrysanthemi.* Said subject might have become hypersensitive to the bacterial asparaginase, resulting in urticaria and/or wheezing.

An asparaginase lacking glutaminase activity for use according to the invention is preferably provided to a patient that is suffering from liver toxicity. Although some abnormalities of liver test results occur in almost all patients treated with asparaginase, a decrease in serum albumin and clotting factors (including II, V, VII, VIII, IX, prothrombin and fibrinogen) may become due to inhibition of hepatic protein synthesis by repeated administration of bacterial asparaginase. Most patients also have a rise in alkaline phosphatase levels and a lower proportion have increases in serum aminotransferase levels and bilirubin. The abnormalities often arise after 2 to 3 weeks of therapy, and are thought to be due to the intrinsic glutaminase activity of bacterial asparaginases (Distasio et al., 1982. Int J Cancer 30, 343-7).

An asparaginase lacking glutaminase activity for use according to the invention may be provided as part of a therapy that further comprises at least one antineoplastic agent. Said at least one antineoplastic agent preferably is selected from vincristine, mercaptopurine, methotrexate, daunorubicin, and prednisone. However, it will be evident to a person skilled in the art that the choice for a specific antineoplastic agent will at least in part be determined by the malignancy of cancer that is to be treated. For example, said asparaginase may be combined with cisplatin and gemcitabine for treatment of a patient suffering from aggressive natural killer cell leukemia (LaPorte et al., 2015. Case Reports in Hematology 2015, 1-3). A combination with gemcitabine or etoposide has been reported to enhance toxicity of glioblastoma cells (Panosyan et al., 2014. Mol Canc Res 12, 694-702). In addition, a combination of asparaginase with navitoclax (4-(4-{[2-(4-Chlorophenyl)-5,5-dimethyl-1-cyclohexen-1-yl]methyl}-1-piperazinyl)-N-[(4-{[(2R)-4-(4-morpholinyl)-1-(phenylsulfanyl)-2-butanyl]amino}-3-[(trifluoromethyl)sulfonyl]phenyl)sulfonyl]benzamide), previously termed ABT-263, has been reported to result in metabolic reprogramming of glioblastoma cells, resulting in induction of apoptosis *in vitro* and in *vivo* (Karpel-Massler et al., 2016. Oncotarget 7, 33512-33528).

The invention further provides a pharmaceutical composition, for use as a medicament, comprising an asparaginase comprising an amino acid sequence that is at least 90% identical to the amino acid sequence depicted in Figure 1 and wherein the asparaginase lacks glutaminase activity, and a pharmaceutically acceptable excipient. Said asparaginase lacking glutaminase activity may be present in the pharmaceutical composition for use as a medicament, as a purified protein, as a pegylated protein as described herein above, or present in a membranous particle as described herein above. Said excipient may be a natural or synthetic substance formulated alongside the active ingredient in the pharmaceutical composition for use as a medicament. An excipient may be included for the purpose of long-term stabilization, reducing viscosity, or enhancing solubility. Suitable excipients include fillers and diluents such as lactose and calcium carbonate, disintegrants such as starch, glycolate and crosscarmellose, binders and adhesives such as polyvinylpyrrolidone and hydroxypropylmethylcellulose, lubricants such as magnesium stearate and stearic acid, and glidants such as talc and silicium oxide (SiO2).

A preferred excipient for use with a membranous particle comprising said asparaginase is artificial cerebrospinal fluid. A suitable artificial cerebrospinal fluid (aCFS) comprises 100-130 mM, preferably 118 or 119 mM, NaCl, 20-30 mM, preferably 26.2 mM NaHCO3, 2-5 mM, preferably 2.5 mM KCl, 03-3 mM, preferably 1 mM NaH2PO4, 0-3 mM, preferably 1.3 mM MgCl2, 0-3 mM CaCl2, preferably 0 mM, and 0-25 mM, preferably 10 mM glucose.

Preferred excipients for use with a purified protein, either pegylated or non-pegylated, are sodium chloride and glucose monohydrate. A preferred dosage of purified protein comprises 2,000-20,000 International Units (IU), preferably 10,000 IU, of asparaginase, 1-10 milligram, preferably about 5 milligram, of glucose monohydrate, and 0.1-1 milligram, preferably about 0.5 milligram, of sodium chloride.

Further described herein is a membranous particle, preferably a vesicle, more preferred a EV, comprising an asparaginase having an amino acid sequence that is at least 90% identical to the amino acid sequence depicted in Figure 1 and wherein the asparaginase lacks glutaminase activity. Said membranous particle is preferably obtained from a human cell, preferably a human stem cell. Methods for producing such membranous particles are known in the art. Examples of such methods are described herein above.

The invention further provides a pharmaceutical composition, for use as a medicament, comprising a membranous particle as described herein above, and a pharmaceutically acceptable excipient. A preferred excipient for a membranous particle such as a cell or a vesicle is a CSF.

### Examples

### Example 1

### Materials and methods

### Cell culture preparations

NSCs were prepared from the subventricular zone (SVZ) of 7- to 12-week-old mice, as described (Cossetti et al., 2014. Mol Cell 56, 193-204). Good Manufacturing Practice (GMP)-grade, foetal human NSCs from natural in utero death (hNSCs) were prepared as described (Mazzini et al., 2015. J Transl Med 13, 17).

### Purification of EVs from media

Mouse and human NSC EVs were collected as described (Cossetti et al., 2014. Mol Cell 56, 193-204).

### Nanoparticle Tracking Analysis (NTA)

Samples were diluted 1:1000 with PBS, vortexed and analysed by Nanoparticle Tracking Analysis (NTA) instrument (NS500, NanoSight ltd) fitted with an Electron Multiplication Charge-Couple Device camera and a 532 nm laser. The temperature was maintained within the range 20-25°C and resultant liquid viscosity calculated by the instrument. Three repeated measurements of 60 seconds were recorded for each of the 3 independent samples (9 analysis in total). Fresh sample was loaded into the chamber prior to each video capture. Static mode (no flow) was used for analysis and detection threshold was adjusted to 5. Automatic settings were set for blur size, minimum track length and minimum particle size. The movement of each particle in the field of view was measured to generate the average displacement (in terms of x and y) of each particle per unit time. From this measurement, the particle diffusion coefficient was estimated and the hydrodynamic diameter calculated through application of the Stokes-Einstein equation.

### Tunable resistive pulse sensing (TRPS)

The concentration and size distribution of EVs was analyzed with TRPS (qNano, Izon Science Ltd) using a NP150 nanopore at 0.5 V with 47 mm stretch. The concentration of particles was standardized using 100 nm calibration beads (CPC 100) at a concentration of 1x1010 particles/mL.

### Protein purification and Western blot analysis

Whole NSC and EV extracts were processed as described (Cossetti et al., 2014. Mol Cell 56, 193-204). The following primary antibodies were used: mouse monoclonal anti-Alix (BD transduction lab and Cell Signaling Technology); rat monoclonal and mouse monoclonal anti-CD9 (BD transduction lab and Life Technologies, respectively); goat polyclonal anti-TSG101 (Santa Cruz); rat monoclonal and mouse monoclonal anti-CD63 (MBL and Life Technologies, respectively); rabbit polyclonal Asrgl1 (Proteintech); rabbit monoclonal Gls (Abcam); mouse monoclonal anti-beta-Actin (Sigma). Molecular weight marker: SeeBlue Plus2 (Invitrogen).

### RNA extraction, RT and qPCR

RNAs from mNSC and hNSC were obtained using the miRCURY^{™} RNA Isolation Kit - Cell & Plant (Exiqon). Total RNA quantity and purity were assessed with the NanoDrop 2000c instrument (Thermo Scientific). cDNA synthesis was performed using SuperScript^{™} III First-Strand Synthesis SuperMix for qRT-PCR (Invitrogen), with random hexamers as primers. qPCRwas performed with the TaqMan^{®} Universal PCR Master Mix (Applied Biosystems) and TaqMan^{®} Gene Expression Assays for FAM^{™} Aspg, Asrgl1, ASPG and ASRGL1 and VIC^{®} euk 18S rRNA probes. Samples were tested in triplicate on a QuantStudio 7 Flex Real-Time PCR System (Applied Biosystems) and analysed with the 2-ΔCt method over 18S ribosomal RNA, used as housekeeping gene.

### Vector production, titration and NSC infection

Lentiviral particles were obtained and used as described (Iraci et al., 2014. Neuromethods 82, 269-288). ShAsrgl1 NSCs were obtained using the pLKO.1-puro third generation vector (Sigma) with the shRNA sequence TRCN32311 (CCGGCCAGAGTTCAACGCAGGTTATCTCGAGATAACCTGCGTTGAACTCTGGTT TTTG) targeting mouse Asrgl1. Asrgl1 Gain of Function (GoF) NSCs were obtained using the pCDH-EF1-MCS-T2A-GFP third generation vector (System Biosciences) with the coding sequence expressing the mouse Asrgl1. A Multiplicity of Infection (MOI) of 30 was used for both shAsrgl1 and Asrgl1 GoF NSCs.

### LC-MS metabolomic analysis.

For the LC separation, column used was the Sequant Zic-Hilic (150mm × 4.6 mm, internal diameter (i.d.) 5 µm) with a guard column (20 mm × 2.1 mm i.d. 5 µm) from HiChrom, Reading, UK. Mobile phase A: 0.1% formic acid v/v in water. Mobile B: 0.1% formic acid v/v in acetonitrile. The flow rate was kept at 300 µL/min and gradient was as follows: 0 minutes 80% of B, 12 minutes 50% of B, 26 minutes 50% of B, 28 minutes 20% of B, 36 minutes 20% of B, 37-45 minutes 80% of B. The mass spectrometer (Thermo Q-Exactive Orbitrap) was operated in a polarity-switching mode. Samples were randomised to avoid machine drifts. Peak intensities were integrated with XCalibur Quan software (Thermo).

### Untargeted metabolomics

DMEM high glucose (Life Technology) was incubated for 24 hours at 37°C and 5% CO2 with or without isolated EVs, corresponding to 100 µg of protein extract. To compare metabolic activities of EVs, conditioned medium (CM) and supernatant (SN), medium was supplemented with 50µM 15N2-L-Asn and 2.5mM 13C5-Gln and incubated for 24 hours at 37°C and 5% CO2 with or without isolated EVs. For all other isotope tracing experiments medium was supplemented with equimolar concentrations of 15N2-L-Asn and 13C5-Gln (50µM). At the end of incubation period metabolites were extracted by diluting 50µL of medium in 750µL extraction buffer (MeOH:ACCN:H2O, 50:30:20). Extracted samples were mixed at 4°C for 15 minutes and proteins were precipitated by centrifugation at 16000g at 4°C for 10 minutes. Supernatant was collected and submitted to LC-MS analysis. To investigate EVs metabolic activity LC-MS peak intensities of fresh medium compared to EVs spent medium were compared.

### Statistical analysis

For untargeted metabolomics analysis statistical analysis was performed with the R package "muma" (https://cran.r-project.org/) by comparing levels of metabolites in fresh and EVs conditioned medium via student's t test. Benjamini-Hochberg correction for multiple testing and FDR=0.05 were applied to determine statistical significance. For isotope tracing experiments statistical analysis was performed with Prism Graphpad and tests were applied as indicated in figure legends.

### Results

### NSCs secrete EVs containing exosomes

We started our investigation by generating EVs from NSCs. Stably expandable NSCs were derived from the subventricular zone (SVZ) of adult mice, and EVs were purified from NSC supernatants by differential centrifugation, as previously described (Cossetti et al., 2014. Mol Cell 56, 193-204). To characterize the population of vesicles secreted by NSCs, we applied a combination of nanoparticle tracking analysis (NTA), tunable resistive pulse sensing (TRPS) technology and Western blot (WB) analysis. NTA identified a major peak (169 ± 3 nm) in the size range of exosomes, and a minor peak (300-500 nm), which is consistent with the dimension of larger shedding vesicles (data not shown). TRPS with a 150 nm nanopore value confirmed the presence of small vesicles, with a major peak corresponding to the size described for exosomes (102 ± 9 nm) (Lobb et al., 2015. J Extracell Vesicles 4, 27031). WB further confirmed that protein extracts purified from EVs were specifically enriched in markers associated with exosomes (vs. parental NSC protein extracts), which include Alix1 (Pdcd6ip), Tsg101 and the tetraspanins CD63 and CD9 (Cossetti et al., 2014. Mol Cell 56, 193-204).

### NSC-derived EVs exhibit L-asparaginase activity

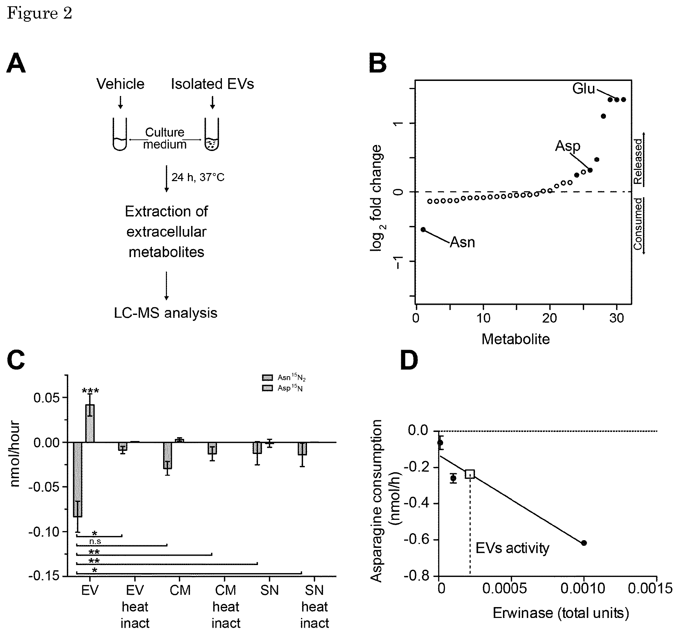

To assess the metabolic activity of NSCs-derived EVs, we incubated EVs in commercial NSC medium and analysed the consumption/release of metabolites from/in the medium via liquid chromatography coupled to mass spectrometry (LC/MS) (Figure 2A). We observed that incubation with EVs induced profound changes in the levels of several metabolites within the medium. In particular, we found that asparagine (Asn) was the most consumed metabolite, whilst aspartate (Asp) and glutamate (Glu) were amongst the most abundantly released (Figure 2B). Given the significant depletion of Asn coupled with the production of Asp, we hypothesised that EVs harbor L-asparaginase activity. To validate this hypothesis, we incubated EVs in commercial NSC medium supplemented with ¹⁵N2-Asn. If EVs carried intact L-asparaginase activity, the resultant Asp should contain one of the two labelled nitrogen of Asn. Consistent with such a hypothesis, we observed marked consumption of ¹⁵N2-Asn coupled with production of ¹⁵N-Asp (Figure 2C). Of note, this metabolic activity was enriched in media containing EVs, as compared to NSC conditioned media (CM) and EV-deprived NSC supernatants (SN). Furthermore, L-asparaginase activity was dampened in heat-inactivated vesicles (Figure 2C), confirming that this activity was in fact dependent on intact enzymatic function. Finally, a calibration curve using clinical-grade L-asparaginase produced by *Erwinia chrysanthemi* (Erwinase) allowed us to quantify the intrinsic L-asparaginase activity of NSCs-derived EVs, giving a value of 2x10-6 U/ µg of EV protein (Figure 2D).

### Mouse and human NSCs traffic Asrgl1 into EVs

We then investigated the identity of the possible enzymes that might confer such asparaginase activity to NSCs EVs. The mouse genome contains two L-asparaginases: a 60 kDa lysophospholipase/L-asparaginase (Aspg) and an isoaspartyl peptidase/L-asparaginase (Asparaginase-like protein 1; Asrgl1) (source http://www.uniprot.org). To assess the expression of Aspg and Asrgl1 in NSCs we took advantage of a comprehensive long RNA-sequence analysis that we recently published (Cossetti et al., 2014. Mol Cell 56, 193-204), and found that NSCs express Asrgl1, but not Aspg. Independent qPCR and WB analyses confirmed the expression of Asgrl1 - but not of Aspg - in NSCs and showed that EVs RNA and protein extracts are enriched in Asrgl1 (Figure 3A-B). We extended our analyses to clinical grade human foetal NSCs (hNSCs) (Mazzini et al., 2015. J Transl Med 13, 17), as well as to EVs collected from hNSC supernatants. Similar to mouse NSCs, hNSCs also expressed ASRGL1 - but not ASPG - and hNSCs-derived EVs were again enriched in ASGRL1 (Figure 3C-D). Thus, EVs from both mouse and human NSCs express Asrgl1, while they do not express Aspg.

### EVs Asrlg1 exhibit L-asparaginase but not glutaminase activity

*In vitro* experiments indicate that Asgrl1 is devoid of activity toward glutamine (Gln) (Cantor et al., 2009. Biochemistry 48, 11026-11031). Yet, its metabolic function within mammalian cells has not been tested. We therefore wanted to investigate Asgrl1 substrate specificity. We first tested whether EVs exhibit any glutaminase (Gls) activity. To this aim, we incubated EVs with ¹³C-Gln and measured the production of ¹³C-Glu, the product of Gls activity (Figure 4A). We could detect Gln consumption by EVs and this was associated with the production of ¹³C-Glu (Figure 4B). Of note, Gln consumption and Glu production by EVs were abolished by heat inactivation, indicating that this activity requires the presence of intact enzymes. In line with this finding we detected protein expression of Gls in EVs (Figure 5A-B).

To assess whether the observed Gls activity could also be linked to Asrgl1 in EVs, we performed loss- (LoF) and gain-of-function (GoF) experiments by transducing NSCs with either a short hairpin RNA targeting Asrgl1 (shAsrgl1) or with the Asrgl1 coding sequence. NSC transduction with LoF and GoF lentiviral vectors did not affect NSC growth and viability upon serial passaging in vitro. LoF and GoF NSCs and EVs showed striking reductions and increases in the levels of Asrgl1 expression respectively, as compared to controls. Of note, these changes in the expression of Asrgl1 had no effects on the level of other endogenous enzymes, such as Gls, both in NSC and EVs (Figure 5A-). We then assessed the relative metabolic activity of EV-trafficked Asrgl1 by incubating control, LoF and GoF EVs in custom made NSC media containing equal amounts (50 µM) of Asn and Gln. Consumption of ¹⁵N2-Asn and production of ¹⁵N-Asp were significantly reduced within media incubated with shAsrgl1 EVs (vs. shCtrl EVs) (Figure 5C). Conversely, significant increases in the consumption of ¹⁵N2-Asn and production of ¹⁵N-Asp within media incubated with Asrgl1 GoF EVs (vs. Ctrl EVs) were noted (Figure 5D). Importantly, incubation of our custom media with both shAsrgl1 EVs and Asrgl1 GoF EVs did not alter the consumption of ¹³C5- Gln or the production of 13C5-Glu (Figure 5C-D) confirming that Asrgl1 activity is specific for Asn and is devoid of Gls activity.

## Claims

1. Asparaginase comprising an amino acid sequence that is at least 90% identical to the amino acid sequence MNPIVVVHGG GAGPISKDRK ERVHQGMVRA ATVGYGILRE GGSAVDAVEG AVVALEDDPE FNAGCGSVLN TNGEVEMDAS IMDGKDLSAG AVSAVQCIAN PIKLARLVME KTPHCFLTDQ GAAQFAAAMG VPEIPGEKLV TERNKKRLEK EKHEKGAQKT DCQKNLGTVG AVALDCKGNV AYATSTGGIV NKMVGRVGDS PCLGAGGYAD NDIGAVSTTG HGESILKVNL ARLTLFHIEQ GKTVEEAADL SLGYMKSRVK GLGGLIVVSK TGDWVAKWTS TSMPWAAAKD GKLHFGIDPD DTTITDLP (Figure 1), for use as a medicament and wherein the asparaginase lacks glutaminase activity.

2. Asparaginase for use according to claim 1, wherein said use is in a method for treating a malignancy, preferably a B cell blood malignancy, or in a method for metabolic reprogramming of a cell, preferably a cancerous cell.

3. Asparaginase for use according to claim 1 or claim 2, wherein said asparaginase is provided in a membranous particle.

4. Asparaginase for use according to claim 3, wherein said membranous particle is a cell, preferably a stem cell.

5. Asparaginase for use according to claim 3, wherein said membranous particle is a liposome or vesicle.

6. Asparaginase for use according to claim 1 or claim 2, wherein said asparaginase is recombinantly expressed in a bacterium, in a yeast, or in a human cell, preferably wherein a nucleotide sequence that encodes said asparaginase is codon-optimized for expression in said bacterium or yeast.

7. Asparaginase for use according to claim 6, wherein the asparaginase is pegylated.

8. Asparaginase for use according to claim 6 or claim 7, wherein said asparaginase is provided in a vector, preferably a viral vector, more preferably a retroviral vector.

9. Asparaginase for use according to any one of claims 1-8, wherein said use is in a method for treatment of a patient suffering from leukemia, myelodysplastic syndrome, or a solid tumor such as hepatocellular carcinoma, renal cell carcinoma, breast cancer, ovarian cancer, brain tumor, or lung cancer.

10. Asparaginase for use according to any one of claims 1-9, wherein said use is in a method for treatment of a patient suffering from acute lymphocytic leukemia or acute myeloid leukemia, especially for treatment of a patient that is suffering from liver toxicity or at risk of developing liver toxicity.

11. Asparaginase for use according to any one of claims 1-10, wherein said asparaginase is provided to a subject that had been treated with an asparaginase from *Escherichia coli* or from *Erwinia chrysanthemi.*

12. Asparaginase for use according to any one of claims 1-11, wherein said asparaginase is provided as part of a therapy that further comprises at least one antineoplastic agent, wherein said at least one antineoplastic agent preferably is selected from vincristine, mercaptopurine, methotrexate, daunorubicin, navitoclax/obatoclax and prednisone.

13. Asparaginase for use according to any one of claims 1-12, wherein the asparaginase is provided in a membranous particle, preferably a cell or a vesicle.

14. A pharmaceutical composition, for use as a medicament comprising an asparaginase comprising an amino acid sequence that is at least 90% identical to the amino acid sequence MNPIVVVHGG GAGPISKDRK ERVHQGMVRA ATVGYGILRE GGSAVDAVEG AVVALEDDPE FNAGCGSVLN TNGEVEMDAS IMDGKDLSAG AVSAVQCIAN PIKLARLVME KTPHCFLTDQ GAAQFAAAMG VPEIPGEKLV TERNKKRLEK EKHEKGAQKT DCQKNLGTVG AVALDCKGNV AYATSTGGIV NKMVGRVGDS PCLGAGGYAD NDIGAVSTTG HGESILKVNL ARLTLFHIEQ GKTVEEAADL SLGYMKSRVK GLGGLIVVSK TGDWVAKWTS TSMPWAAAKD GKLHFGIDPD DTTITDLP (Figure 1), wherein the asparaginase lacks glutaminase activity, or comprising a membranous particle comprising said asparaginase as defined in claim13, and a pharmaceutically acceptable excipient.

15. The pharmaceutical composition according to claim 14, wherein the excipient comprises an artificial cerebrospinal fluid.

## Patentansprüche

1. Asparaginase, umfassend eine Aminosäuresequenz, die mindestens 90 % identisch ist mit der Aminosäuresequenz MNPIVVVHGG GAGPISKDRK ERVHQGMVRA ATVGYGILRE GGSAVDAVEG AVVALEDDPE FNAGCGSVLN TNGEVEMDAS IMDGKDLSAG AVSAVQCIAN PIKLARLVME KTPHCFLTDQ GAAQFAAAMG VPEIPGEKLV TERNKKRLEK EKHEKGAQKT DCQKNLGTVG AVALDCKGNV AYATSTGGIV NKMVGRVGDS PCLGAGGYAD NDIGAVSTTG HGESILKVNL ARLTLFHIEQ GKTVEEAADL SLGYMKSRVK GLGGLIVVSK TGDWVAKWTS TSMPWAAAKD GKLHFGIDPD DTTITDLP (Figur 1) zur Verwendung als ein Arzneimittel, und wobei der Asparaginase Glutaminase-Aktivität fehlt.

2. Asparaginase zur Verwendung nach Anspruch 1, wobei die Verwendung in einem Verfahren zur Behandlung einer Malignität, vorzugsweise einer Malignität der B-Zellen des Blutes, oder in einem Verfahren zum metabolischen Umprogrammieren einer Zelle, vorzugsweise einer Krebszelle, ist.

3. Asparaginase zur Verwendung nach Anspruch 1 oder Anspruch 2, wobei die Asparaginase in einem Membranpartikel bereitgestellt ist.

4. Asparaginase zur Verwendung nach Anspruch 3, wobei der Membranpartikel eine Zelle, vorzugsweise eine Stammzelle ist.

5. Asparaginase zur Verwendung nach Anspruch 3, wobei der Membranpartikel ein Liposom oder ein Vesikel ist.

6. Asparaginase zur Verwendung nach Anspruch 1 oder Anspruch 2, wobei die Asparaginase rekombinant in einem Bakterium, in einer Hefe oder in einer menschlichen Zelle exprimiert ist, wobei vorzugsweise eine Nukleotidsequenz, die die Asparaginase kodiert, für die Expression in dem Bakterium oder der Hefe codon-optimiert ist.

7. Asparaginase zur Verwendung nach Anspruch 6, wobei die Asparaginase pegyliert ist.

8. Asparaginase zur Verwendung nach Anspruch 6 oder Anspruch 7, wobei die Asparaginase in einem Vektor, bevorzugt einem viralen Vektor, bevorzugter einem retroviralen Vektor bereitgestellt ist.

9. Asparaginase zur Verwendung nach einem der Ansprüche 1 bis 8, wobei die Verwendung in einem Verfahren zur Behandlung eines Patienten, der an Leukämie, myelodysplastischem Syndrom, oder einem soliden Tumor wie einem hepatozellulären Karzinom, Nierenzellkarzinom, Brustkrebs, Ovarialkarzinom, Hirntumor oder Lungenkrebs leidet, ist.

10. Asparaginase zur Verwendung nach einem der Ansprüche 1-9, wobei die Verwendung in einem Verfahren zur Behandlung eines Patienten, der an einer akuten lymphatischen Leukämie oder akuten myeloischen Leukämie leidet, insbesondere zur Behandlung eines Patienten, der an einer Lebertoxizität oder dem Risiko der Entwicklung einer Lebertoxizität leidet, ist.

11. Asparaginase zur Verwendung nach einem der Ansprüche 1 bis 10, wobei die Asparaginase einem Subjekt zur Verfügung gestellt wird, das mit einer Asparaginase von *Escherichia coli* oder von *Erwinia chrysanthemi* behandelt wurde.

12. Asparaginase zur Verwendung nach einem der Ansprüche 1-11, wobei die Asparaginase als Teil einer Therapie zur Verfügung gestellt wird, die ferner wenigstens einen antineoplastischen Wirkstoff umfasst, wobei der wenigstens eine antineoplastische Wirkstoff vorzugsweise ausgewählt ist aus Vincristin, Mercaptopurin, Methotrexat, Daunorubicin, Navitoclax/Obatoclax und Prednison.

13. Asparaginase zur Verwendung nach einem der Ansprüche 1-12, wobei die Asparaginase in einem Membranpartikel, vorzugsweise einer Zelle oder einem Vesikel, zur Verfügung gestellt ist.

14. Pharmazeutische Zusammensetzung zur Verwendung als ein Arzneimittel, umfassend eine Asparaginase, umfassend eine Aminosäuresequenz, die mindestens 90 % identisch ist mit der Aminosäuresequenz MNPIVVVHGG GAGPISKDRK ERVHQGMVRA ATVGYGILRE GGSAVDAVEG AVVALEDDPE FNAGCGSVLN TNGEVEMDAS IMDGKDLSAG AVSAVQCIAN PIKLARLVME KTPHCFLTDQ GAAQFAAAMG VPEIPGEKLV TERNKKRLEK EKHEKGAQKT DCQKNLGTVG AVALDCKGNV AYATSTGGIV NKMVGRVGDS PCLGAGGYAD NDIGAVSTTG HGESILKVNL ARLTLFHIEQ GKTVEEAADL SLGYMKSRVK GLGGLIVVSK TGDWVAKWTS TSMPWAAAKD GKLHFGIDPD DTTITDLP (Figur 1), wobei der Asparaginase Glutaminase-Aktivität fehlt, oder umfassend ein Membranpartikel, umfassend die Asparaginase wie in Anspruch 13 definiert und einen pharmazeutisch akzeptablen Träger.

15. Pharmazeutische Zusammensetzung nach Anspruch 14, wobei der Träger eine künstliche Liquorflüssigkeit umfasst.

## Revendications

1. Asparaginase comprenant une séquence d'acides aminés qui est identique à au moins 90 % à la séquence d'acides aminés 
MNPIVVVHGG GAGPISKDRK
ERVHQGMVRA ATVGYGILRE GGSAVDAVEG AVVALEDDPE FNAGCGSVLN TNGEVEMDAS
IMDGKDLSAG AVSAVQCIAN PIKLARLVME KTPHCFLTDQ GAAQFAAAMG VPEIPGEKLV
TERNKKRLEK EKHEKGAQKT DCQKNLGTVG AVALDCKGNV AYATSTGGIV NKMVGRVGDS
PCLGAGGYAD NDIGAVSTTG HGESILKVNL ARLTLFHIEQ GKTVEEAADL SLGYMKSRVK
GLGGLIVVSK TGDWVAKWTS TSMPWAAAKD GKLHFGIDPD DTTITDLP (Figure 1),
 pour l'utilisation en tant que médicament et où l'asparaginase est exempte d'activité de glutaminase.

2. Asparaginase pour l'utilisation selon la revendication 1, dans laquelle ladite utilisation est dans une méthode de traitement d'une malignité, de préférence d'une hémopathie maligne à lymphocytes B, ou dans une méthode de reprogrammation métabolique d'une cellule, de préférence d'une cellule cancéreuse.

3. Asparaginase pour l'utilisation selon la revendication 1 ou la revendication 2, où ladite asparaginase est fournie dans une particule membranaire.

4. Asparaginase pour l'utilisation selon la revendication 3, dans laquelle ladite particule membranaire est une cellule, de préférence une cellule souche.

5. Asparaginase pour l'utilisation selon la revendication 3, dans laquelle ladite particule membranaire est un liposome ou une vésicule.

6. Asparaginase pour l'utilisation selon la revendication 1 ou la revendication 2, où ladite asparaginase est exprimée de manière recombinante dans une bactérie, dans une levure, ou dans une cellule humaine, de préférence dans laquelle une séquence nucléotidique qui code pour ladite asparaginase a été soumise à une optimisation de codon pour l'expression dans ladite bactérie ou levure.

7. Asparaginase pour l'utilisation selon la revendication 6, où ladite asparaginase est pégylée.

8. Asparaginase pour l'utilisation selon la revendication 6 ou la revendication 7, où ladite asparaginase est fournie dans un vecteur, de préférence un vecteur viral, de manière mieux préférée dans un vecteur rétroviral.

9. Asparaginase pour l'utilisation selon l'une quelconque des revendications 1 à 8, dans laquelle ladite utilisation est dans une méthode de traitement d'un patient souffrant de leucémie, du syndrome myélodysplasique ou d'une tumeur solide comme un carcinome hépatocellulaire, un carcinome à cellules rénales, un cancer du sein, un cancer des ovaires, une tumeur au cerveau ou un cancer du poumon.

10. Asparaginase pour l'utilisation selon l'une quelconque des revendications 1 à 9, dans laquelle ladite utilisation est dans une méthode de traitement d'un patient souffrant de leucémie lymphoblastique aiguë ou de leucémie myéloïde aiguë, en particulier pour le traitement d'un patient qui est atteint d'une toxicité hépatique ou qui risque de développer une toxicité hépatique.

11. Asparaginase pour l'utilisation selon l'une quelconque des revendications 1 à 10, où ladite asparaginase est fournie à un sujet qui a été traité par une asparaginase provenant d*'Escherichia coli* ou d*'Erwinia chrysanthemi.*

12. Asparaginase pour l'utilisation selon l'une quelconque des revendications 1 à 11, où ladite asparaginase est fournie en tant que partie d'une thérapie qui comprend en outre au moins un agent anti-néoplasique, où ledit au moins un agent anti-néoplasique est de préférence choisi parmi la vincristine, la mercaptopurine, le méthotrexate, la daunorubicine, le navitoclax/l'obatoclax et la prednisone.

13. Asparaginase pour l'utilisation selon l'une quelconque des revendications 1 à 12, où l'asparaginase est fournie dans une particule membranaire, de préférence une cellule ou une vésicule.

14. Composition pharmaceutique pour l'utilisation en tant que médicament comprenant une asparaginase comprenant une séquence d'acides aminés qui est identique à au moins 90 % à la séquence d'acides aminés MNPIVVVHGG GAGPISKDRK ERVHQGMVRA ATVGYGILRE GGSAVDAVEG AVVALEDDPE FNAGCGSVLN TNGEVEMDAS IMDGKDLSAG AVSAVQCIAN PIKLARLVME KTPHCFLTDQ GAAQFAAAMG VPEIPGEKLV TERNKKRLEK EKHEKGAQKT DCQKNLGTVG AVALDCKGNV AYATSTGGIV NKMVGRVGDS PCLGAGGYAD NDIGAVSTTG HGESILKVNL ARLTLFHIEQ GKTVEEAADL SLGYMKSRVK GLGGLIVVSK TGDWVAKWTS TSMPWAAAKD GKLHFGIDPD DTTITDLP (Figure 1),
dans laquelle l'asparaginase est exempte d'activité de glutaminase, ou comprenant une particule membranaire comprenant ladite asparaginase telle que définie selon la revendication 13, et un excipient pharmaceutiquement acceptable.

15. Composition pharmaceutique selon la revendication 14, dans laquelle l'excipient comprend un liquide céphalo-rachidien artificiel.
